(19) Europäisches Patentamt
European Patent Office
Office européen des brevets

(11) Numéro de publication: **0 041 410**
**B1**

(12) # FASCICULE DE BREVET EUROPEEN

(45) Date de publication du fascicule du brevet: **09.11.83**

(21) Numéro de dépôt: **81400551.8**

(22) Date de dépôt: **07.04.81**

(51) Int. Cl.³: **C 07 D 321/10**, C 07 D 327/02, C 07 D 405/06, C 07 D 409/06, A 61 K 31/335, A 61 K 31/38, A 61 K 31/445, A 61 K 31/40, A 61 K 31/495 // C07C121/75, C07C65/21

(54) **Nouveaux éthers tricycliques, leurs procédés de préparation et leur utilisation comme médicament.**

(30) Priorité: **10.04.80 FR 8008023**

(43) Date de publication de la demande: **09.12.81 Bulletin 81/49**

(45) Mention de la délivrance du brevet: **09.11.83 Bulletin 83/45**

(84) Etats contractants désignés: **AT BE CH DE GB IT LI NL SE**

(56) Documents cités:
**FR - A - 1 312 889**
**FR - A - 1 312 891**
**FR - A - 2 315 272**
**FR - M - 1 827**

**Burger's Medicinal Chemistry, Part III, pp 1030-1031**

**Le dossier contient des informations techniques présentées postérieurement au dépôt de la demande et ne figurant pas dans le présent fascicule.**

(73) Titulaire: **SCIENCE UNION ET Cie SOCIETE FRANCAISE DE RECHERCHE MEDICALE, 14 rue du Val d'Or, F-92150 Suresnes (FR)**

(72) Inventeur: **Malen, Charles, 3 allée Traversière, F-94260 Fresnes (FR)**
Inventeur: **Poignant, Jean-Claude, Dr., Rue de la Fontaine St-Mathieu, F-91440 Bures s/Yvette (FR)**

(74) Mandataire: **Varady, Peter et al, Service Brevets 22 rue Garnier, F-92200 Neuilly sur Seine (FR)**

EP 0 041 410 B1

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

BUNDESDRUCKEREI BERLIN

# Nouveaux éthers tricycliques, leurs procédés de préparation et leur utilisation comme médicament

La présente invention a pour objet de nouveaux éthers tricycliques, leurs procédés de préparation et leur utilisation comme médicament.

On connait des composés tricycliques portant une chaîne latérale amino-alkyle, notamment amino-propyle, actifs sur le système nerveux central, comme antidépresseurs, en particulier l'imipramine et d'autres composés de ce type (voir Burger's Medicinal Chemistry, fourth edition, part III, p. 1030–31).

On a maintenant trouvé une nouvelle famille de composés ayant un effet antidépresseur exceptionnel, et un effet sédatif très faible.

Spécifiquement l'invention se rapporte à des dibenzo oxépines substituées par une chaîne amino-éthyle répondant à la formule générale I

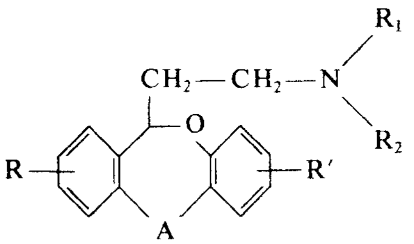

(I)

dans laquelle R et R' identiques ou différents, représentent un atome d'hydrogène ou d'halogène, ou bien un groupe trifluorométhyle,

A représente un atome d'oxygène ou de soufre,

$R_1$ et $R_2$, identiques ou différents, représentent un atome d'hydrogène ou un groupe alkyle de 1 à 4 atomes de carbone ou bien forment ensemble un groupe pyrrolidino.

L'invention concerne aussi les sels d'addition avec un acide minéral ou organique des composés de formule générale I. On utilise de préférence un acide thérapeutiquement compatible. Toutefois les autres acides peuvent servir de moyen d'identification, d'isolement, de purification ou de dédoublement.

Les composés de formule générale I possèdent un ou plusieurs centres de chiralité et de ce fait peuvent être dédoublés en leurs isomères optiques. Les formes dextrogyre et lévogyre font partie de l'invention au même titre que les composés racémiques.

Parmi les acides que l'on peut ajouter aux composés de formule générale I pour former un sel d'addition on pourra citer plus particulièrement, et à titre d'exemples non limitatifs, l'acide chlorhydrique, l'acide bromhydrique, l'acide nitrique, l'acide phosphorique, l'acide hypophosphorique, l'acide sulfurique, l'acide sulfureux, l'acide thiosulfurique, l'acide métaphosphorique, l'acide formique, l'acide acétique, l'acide lactique, l'acide pyruvique, l'acide 1-cétogulonique, l'acide lévulinique, l'acide succinique, l'acide tartrique, l'acide citrique, l'acide fumarique, l'acide maléique, l'acide itaconique, l'acide caproïque , l'acide benzoïque- l'acide salicylique, l'acide dichlorobenzoïque, l'acide vératrique, l'acide syringique, l'acide eugénique, l'acide isonicotinique, l'acide thiazole 5-carboxylique, l'acide méthane sulfonique l'acide iséthionique, l'acide benzène sulfonique, l'acide glucose 1-phosphorique, l'acide glucose 1,6-diphosphorique ou l'acide adénosine diphosphorique.

Les composés de formule générale I sont doués de propriétés pharmacologiques intéressantes. Ce sont des substances psychotropes qui manifestent sur le système nerveux central des effets antidépresseurs et anxiolytiques. Sur l'animal ils entrainent une diminution de tonus musculaire, une inhibition de l'aggressivité et ont un effet anticonvulsivant, anticatatonique et antihypothermique.

Par ailleurs, ils antagonisent la ptose des paupières provoquée par la tétrabenazine et ils augmentent le nombre des mouvements stéréotypés provoquée par l'amphétamine.

En outre, ils sont actifs aussi bien par voie buccale que par voie parentérale.

Ils trouvent donc emploi en thérapeutique humaine ou animale comme médicaments anti-dépresseurs ou anxiolytiques. Ils seront utilisables pour le traitement des états dépressifs isolés ou associés à l'anxiété.

Chez l'enfant ils trouvent emploi pour le traitement des difficultés scolaires associées à la dépression.

L'invention a aussi pour objet les compositions pharmaceutiques renfermant au moins un composé de formule générale I ou un de ses sels d'addition avec un acide minéral ou organique à titre de principe actif, en association ou en mélange avec un ou plusieurs excipients ou véhicules inertes non-toxiques, pharmaceutiquement acceptables.

Parmi les compositions pharmaceutiques selon l'invention on pourra citer plus particulièrement celles qui conviennent pour l'administration par voie parentérale, par voie buccale, par voie rectale ou par voie sublinguale et notamment les comprimés nus, enrobés ou à délitement retardé, les gélules, les microcapsules, les pilules, les suspensions, solutions ou émulsions buvables, les gouttes, les

solutés ou suspensions injectables conditionnés en ampoules, en flacons multidoses ou en seringues auto-injectables; les suppositoires; les comprimés sublinguaux.

La posologie utile varie selon l'age et le poids du patient, la voie d'administration et la nature de l'indication thérapeutique.

D'une manière générale la posologie unitaire s'étend de 0,5 mg à 10 mg et la posologie journalière de 0,5 à 30 mg chez l'homme. En médecine vétérinaire la posologie sera ajustée au poids de l'animal. Ches l'enfant la posologie pourra être sensiblement diminuée en fonction du poids du sujet.

L'invention a aussi pour objet les procédés de préparation des dibenzo oxépines de formule générale I, caractérisés en ce que l'on soumet un acide (hydroxyphényl) A-benzoïque de formule générale II de la planche des formules

dans laquelle les substituants R, et R' ont les significations fournies antérieurement,

et A est die l'oxygène ou du soufre, à l'action d'un agent déshydratant tel que l'anhydride acétique ou à l'action de chlorure de thionyle et à la cyclisation par une amine tertiaire pour former une dibenzo oxépinone de formule générale III dans laquelle la signification des substituants R, R', et A, demeure inchangée

que l'on soumet à l'action sélective d'un ylide d'alcoxycarbonylméthyl) phosphonium de formule IV

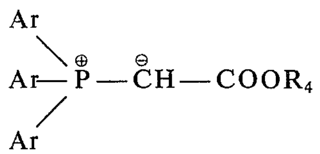
(IV)

dans laquelle Ar représente un radical phényle ou phényle substitué

et $R_4$ est un radical alcoyle inférieur ou aralcoyle substitué ou non, pour former le dérivé (alcoxycarbonyl) méthylidénique correspondant de formule générale V

dans laquelle les substituants A, R, R' et A gardent les significations fournies ci-dessus, et

on soumet celui-ci à l'action d'un agent d'hydrogénation pour former un dérivé (alcoxycarbonyl) méthylénique de formule générale VI

dans laquelle les substituants R, R', $R_4$ et A ont les mêmes définitions que ci-dessus, et

on réduit ce dernier en éthanol correspondant par action d'un agent d'hydrogénation en particulier, d'un hydrure mixte de métal alcalin pour obtenir un composé de formule générale VII de la planche de formules

dans laquelle R, R', et A sont définis comme précédemment et l'on transforme ce dérivé en ester de formule VIII tels que chlorure, iodure, bromure, alkyl ou arylsulfonate par des procédés connus

dans laquelle la définition des substituants R, R', et A demeure inchangée

et X est Cl, Br, I, alkyl $SO_3$ ou aryl $SO_3$

puis fait réagir celui-ci avec un dérivé aminé de formule générale IX

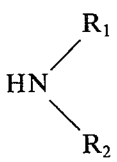
(IX)

dans laquelle $R_1$ et $R_2$ ont les définitions fournies précédemment

et obtient un dérivé éthylaminé de formule générale I dans laquelle la définition des substituants R, R', $R_1$, $R_2$ et A demeure inchangée,

que l'on peut salifier par addition d'un acide minéral ou organique.

L'invention s'étend également à un procédé d'obtention des composés de formule générale I, procédé qui est caractérisé en ce que l'on soumet une dibenzo-oxépinone de formule générale III

dans laquelle R, R', et A sont définis comme précédemment à l'action d'un ylide de cyanométhyl phosphonium de formule générale IV

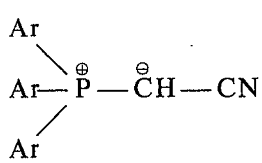
(IV')

dans laquelle Ar est défini comme précédemment pour former un dérivé cyanométhylidénique de formule générale XI

dans laquelle R, R', et A sont définis comme précédemment que l'on hydrogène sélectivement en dérivés amino éthylidéniques correspondant de formule générale XII

sous forme Z ou E

dans laquelle les substituants A, R, et R' sont définis comme ci-dessus puis réduit ces derniers à l'aide d'un agent d'hydrogénation tel qu'un hydrure métallique ou hydrogénation catalytique pour former un dérivé éthylaminé de formule I dans laquelle $R_1$ et $R_2 = H$
que l'on peut:
soit salifier par addition d'un acide minéral ou organique
soit dédoubler en ses isomères optiques par salification à l'aide d'un acide chiral
soit alcoyler ou aralcoyler par action d'un dérivé carbonylé en présence d'un agent réducteur.

L'invention a encore pour objet un procédé d'obtention des composés de formule générale I qui consiste à soumettre un composé alcoyloxycarbonylméthylidénique de formule générale V à une saponification ménagée en milieu alcalin pour former un dérivé (hydroxycarbonyl) méthylidénique de formule générale XIV
dans laquelle les substituants R, R' et A ont les significations fournies antérieurement, sous forme Z ou E
que l'on condense avec un dérivé aminé de formule générale IX dans laquelle les substituants $R_1$ et $R_2$ ont les significations fournies précédemment, en présence d'un agent de fonctionnalisation du carboxyle pour former l'amide méthylidénique correpondant de formule générale XV dans laquelle les substituants $R_1$, $R_2$, R, R', et A ont les significations fournies antérieurement
que l'on peut réduire ou hydrogéner en dérivé éthylaminé de formule générale I.

Les exemples suivants illustrent l'invention. Ils ne la limitent en aucune façon. Les planches de formules jointes explicitent le schéma réactionnel et les différentes structures des composés produits.

## Exemple 1

### dl N-diéthyl 2-[dibenzo (b,e) 1,4-dioxépin-11-yl] éthylamine et son fumarate

### Stade A

### dibenzo (b,e) 1,4-dioxépine 11-one

On met en suspension 5,06 g d'acide (2-hydroxyphénoxy) 2-benzoïque dans 35 ml d'anhydride acétique fraichement redistillé puis on porte au reflux le mélange pendant 3 heures en suivant le déroulement de la réaction par analyse d'un échantillon en CPV. On chasse ensuite par distillation sous pression réduite l'excès d'anhydride acétique et on recueille un résidu huileux qui cristallise par repos. Après une nuit en glacière on reprend les cristaux par quelques ml d'éther. On sépare la masse cristalline par filtration, on essore, on rince avec un peu d'éther et on sèche sous vide. La dibenzo (b,e) dioxépinone est purifiée par distillation fractionnée sous pression réduite, le produit pur distille à 140 – 145° sous 0,01 mm Hg et fond à 62 – 66°.

### Stade B

### 11-(méthoxycarbonyl) méthylidène [dibenzo (b,e) 1,4-dioxépine]

On dissout 15,90 g de dibenzo (b,e) dioxépine 11-one dans 25 ml de toluène puis on ajoute 25 g d'ylide de (méthoxy carbonyl méthylène) triphényl phosphonium. On chauffe le mélange au reflux pendant 15 heures. On laisse ensuite refroidir et on ajoute à nouveau 2,5 g d'ylide de phosphonium. On poursuit le chauffage au reflux pendant encore 24 heures jusqu'à ce que la CPV indique une absence du produit de départ.

On arrête le chauffage puis on chasse le solvant sous vide. On reprend le résidu par de l'éther anhydre. Il se forme une masse gommeuse que l'on abandonne. L'éther est filtré puis évaporé à sec. On obtient ainsi 33 g de produit brut formé d'un mélange d'isomères Z et E.

On purifie le produit par chromatographie sur colonne de silice. On élue avec un mélange benzène chloroforme à parties égales et on prélève des fractions de 50 ml. Après élimination des fractions de tête on recueille une fraction riche en isomère A. Après recristallisation de l'acétonitrile à reflux par chaud et froid on obtient 2,7 g de l'isomère A pur fondant à 59 – 60°.

Des fractions de queue on isole une huile qui cristallise lentement; par recristallisation de l'éther isopropylique on recueille 4 g de l'isomère B fondant à 81 – 83°.

### Stade C

### 11-(méthoxycarbonyl méthyl) [dibenzo (b,e) 1,4-dioxépine]

On dissout dans 150 ml de méthanol, 27 g de 11-(méthoxy carbonyl méthylidène) dibenzo (b,e)

1,4-dioxépine (isomère A ou isomère B). On purge la solution par barbottage d'azote puis on ajoute 0,5 g de dioxyde de platine et on procède à l'hydrogénation sous pression atmosphérique jusqu'à cessation d'absorption. On filtre alors le catalyseur puis concentre à demi-volume la solution méthanolique. On amorce la cristallisation puis laisse reposer le mélange cristallin pendant 10 heures en glacière. On essore la masse cristalline, puis la sèche sous vide phosphorique. On obtient ainsi 13 g de produit hydrogéné soit un rendement de 48%. Le produit pur après recristallisation fond à 72—74°.

La même réduction peut être effectuée par l'amalgame d'aluminium.

L'amalgame d'aluminium préparé à partir de 50,3 g d'aluminium et 24,7 g de chlorure mercurique est mis en suspension dans 680 ml d'éther sec. On ajoute alors la solution de 75 g de 11 (méthoxy carbonyl méthylidène) dibenzo (b,e) 1,4-dioxépine (isomère A ou B) dans 50 ml d'éther sec. On additionne sous bonne agitation pendant 4 heures la solution de 23,5 ml d'eau dans 25 ml d'acétonitrile. Le mélange réactionnel est porté spontanément à léger reflux. L'addition d'eau terminée, la phase organique isolée, lavée, séchée est évaporée sous vide. Le résidu sec 60,8 g est recristallisé dans l'éthanol (100 ml)

Rendement: 38 g 51% F=72—75° MK

Stade D

11-hydroxyéthyl [dibenzo (b,e) 1,4-dioxépine]

On charge 33,7 g de 11-(méthoxycarbonylméthyl) dibenzo (b,e) 1,4-oxépine dans 50 ml de tétrahydrofuran. On refroidit entre 0 et 10° la solution limpide et on ajoute très lentement 270 ml d'une solution normale de triéthylborohydrure de lithium dans le tétrahydrofuran. La durée de l'addition du réactif est de 6 heures environ. On maintient l'agitation et le refroidissement pendant toute cette période. On laisse ensuite une heure à 0—5° puis on hydrolyse l'excès de réactif par addition de 100 ml d'acide chlorhydrique 3N. On évapore ensuite le tétrahydrofuran. On extrait la phase aqueuse résiduelle à l'éther isopropyl à trois reprises. Les solutions organiques sont rénuies, lavées à l'acide chlorhydrique puis à l'eau. On distille le solvant et on obtient 32,2 g de 11-hydroxyéthyl dibenzo (b,e) 1,4-dioxépine. Le produit est recristallisé du tétrahydrofuran pour recueillir 19,6 g de produit pur soit un rendement de 65%. Le dérivé hydroxéthylé distille à 160—163° sous 0,1 mm Hg.

F=62—64° MK.

Stade E

11-(p.tolylsulfonyloxyéthyl) [dibenzo (b,e) 1,4-dioxépine]

On dissout 7,4 g de dérivé 11-hydroxyéthylé dans 4,8 ml de pyridine et 20 ml d'acétonitrile. On ajoute progressivement une solution de 6,1 g de chlorure de para toluène sulfonyle dans 20 ml d'acétonitrile en 30 mn environ. On laisse le mélange sous agitation à température ordinaire pendant 12 heures puis on sépare par filtration le chlorhydrate de pyridine qui s'est formé. On évapore ensuite l'acétonitrile du filtrat et reprend le résidu par l'eau et l'éther. On sépare la phase éthérée qu'on lave à l'acide sulfurique normal puis à l'eau jusqu'à neutralité des eaux de lavage. On sèche l' ether sur sulfate de magnésium puis évapore à sec. On obtient ainsi 11 g d'un produit huileux que l'on purifie par cristallisation dans 15 ml d'éther isopropylique. On laisse reposer 24 h en glacière puis filtre les cristaux que l'on sèche sous vide.

On obtient ainsi 8,7 g de 11-(p.toluène sulfonyloxyéthyl) dibenzo (b,e) 1,4-dioxépine soit un rendement de 73%. Le produit pur fond à 64—68°.

Stade F

N-diéthyl 2[dibenzo (b,e) 1,4-dioxépin-11 yl] éthylamine

Dans 100 ml d'acétonitrile on dissout 13,2 g de 11-(p.toluène sulfonyloxyéthyl) dibenzo (b,e)-1,4-dioxépine et 4 g de diéthylamine. On porte à 90° pendant une heure puis on laisse refroidir. Le mélange réactionnel est versé dans un mélange d'eau et de glace puis on extrait le précipité au chlorure de méthylène. On sépare la phase organique qu'on sèche puis évapore sous vide. Le résidu sec est mis en suspension dans 20 ml d'éther et 15 ml d'eau. On alcalinise avec 15 ml de soude normale. On agite vigoureusement puis sépare la phase éthérée. La phase éthérée est acidifiée par addition d'acide chlorhydrique puis on alcanise la phase aqueuse. Le précipité apparu est à l'éther. La phase éthérée est lavée à l'eau, séchée sur sulfate de sodium, filtrée et amenée à sec. On recueille

ainsi 9,67 g de dérivé diéthylaminé.

Stade G

Citrate de N-diéthyl 2[dibenzo (b,e) 1,4-dioxépin 11-yl] ethylamine

On met en suspension dans 5 ml d'éthanol 1,2 g de N-diéthyl dibenzo (b,e) 1,4-dioxépin 11-yl ethylamine puis 0,81 g d'acide citrique en solution dans 15 ml d'éthanol chaud. On mélange les réactifs et on concentre à demi-volume la solution claire formée. On amorce la cristallisation et on laisse reposer une nuit à température ordinaire. On sépare le précipité de citrate que l'on rince avec 2 ml d'éthanol puis sèche à 50° sosus vide. On obtient ainsi 1,2 g de citrate de N-diéthyl 2-[dibenzo (b,e) 1,4-dioxépin 11-yl] éthylamine fondant à 123–127°. Ce sel est insoluble dans l'eau.

Selon le même procédé on prépare le fumarate de N-diéthyl 2-[dibenzo (b,e) 1,4-dioxépin 11-yl] éthylamine.

F = 146 – 148° (isopropanol).

Exemple 2

N-diéthyl 2-[2-chloro dibenzo (b,e) 1,4-dioxépin 11-yl] éthylamine et son fumarate

En opérant selon le mode opératoire de l'exemple 1 au départ de l'acide (2'-hydrophénoxy) 2–5 chlorobenzoïque (F = 120 – 121°) on obtient successivement:

— la 2-chloro dibenzo (b,e) 1,4-dioxépine 11-one
 F = 155 – 158° (sublim)
— la 11-(méthoxycarbonylméthylidène) 2-chloro[dibenzo (b,e) 1,4-dioxépine]isomères Z et E
 F = 123 – 126° et F = 102 – 106°
— la 11-(methoxy carbonyl méthyl) [2-chloro dibenzo (b,e) 1,4-dioxépine]
 sous forme d'un produit huileux
— la 11-hydroxyéthyl [2-chloro dibenzo (b,e) 1,4-dioxépine] sous forme d'un produit huileux
— la 11-(p.toluène sulfonyloxyéthyl) [2-chloro dibenzo (b,e) 1,4-dioxépine]
 F = 100 – 108° (rendement 50%)
— la N-diéthyl [2-chlorodibenzo (b,e) 1,4-dioxépine 11-yl] éthylamine
— le fumarate de N-diéthyl 2[2-chlorodibenzo (b,e) 1,4-dioxépin-11-yl] éthylamine
 F = 154 – 158° (acétonitrile)

Exemple 3

dl N-diéthyl 2-[8-chlorodibenzo (b,e) 1,4-dioxépin 11-yl] éthylamine et son chlorhydrate

En opérant selon le mode opératoire de l'exemple 1 au départ de l'acide (2'-hydroxy 4'-chloro) 2-phénoxybenzoïque on obtient successivement:

— la 8-chlorodibenzo (b,e) 1,4-oxépine 11-one
— la 11-(méthoxycarbonylméthylidène) 8-chloro [dibenzo (b,e) 1,4-dioxépine]
 sous forme d'isomères Z et E
— la 11-(méthoxycarbonylméthyl) 8-chloro [dibenzo (b,e) 1,4-dioxépine]
 sous forme d'un produit huileux
— la 11-hydroxyéthyl 3-chloro [dibenzo (b,e) 1,4-dioxépine]
— la 11-(p.toluène sulfonyloxyéthyl) 8-chloro [dibenzo (b,e) 1,4-dioxépine]
 F = 90 – 92°
— la N-diéthyl [(8-chlorodibenzo (b,e) 1,4-dioxépin 11-yl] éthylamine
— la chlorhydrate de N-diéthyl [(8-chloro dibenzo (b,e) 1,4-dioxépin 11-yl)] éthylamine
 F = 150 – 154°

Exemple 4

N-diméthyl 2-[2-trifluorométhyldibenzo (b,e) 1,4-dioxépin 11- l] éthylamine et son fumarate

— la 2 trifluorométhyl dibenzo (b,e) 1,4-oxépine 11-one obtenu par la suite des réactions suivantes:

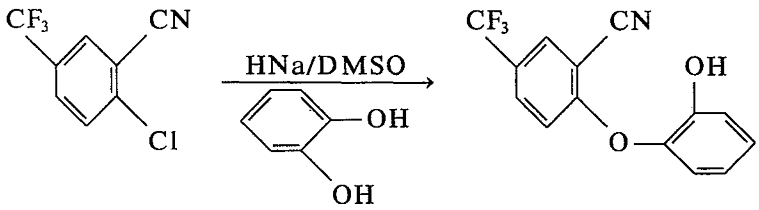


(I)

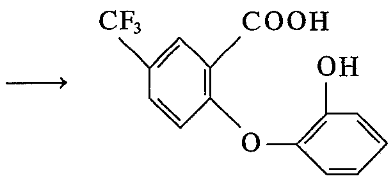

(II)

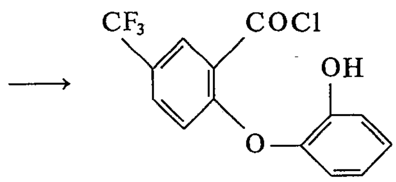

(III)

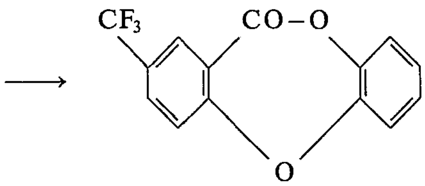

(IV)

1. En atmosphère inerte, on prépare une solution de monosel de sodium de la pyrcatéchine dans le DMSO à partir de 1 mol pyrocatéchine et 0,5 mol d'hydrure de sodium dans 350 ml de DMSO anhydre.

On coule en 7 h. dans cette solution, la solution de 0,5 mol de chloro-2 trifluorométhyl-5 benzonitrile dans 300 ml de DMSO anhydre.

Après la fin de la coulée on porte 5h. à 60°.

Le DMSO est évaporé sous vide. Le résidu est repris par l'eau et acidifié. On extrait le produit à l'éther. Le solvant est évaporé et le résidu cristallisé dans cyclohexane-benzène 70/30.

Rt=74,5 g F=115–118° 53%

2. 83,7 g du nitrile précédent sont additionnés à 315 ml NaOH aq. 40%.

Le mélange réactionnel est reflué 4 h, puis dilué par de la glace, acidifié et extrait à l'éth; après évaporation du solvant, le résidu huileux 87 g (97%) est utilisé brut pour la réaction suivante.

3. 96,6 g de l'acide précédent en solution dans 400 ml de benzène sec sont traités par 154 g de chlorure de thionyle à reflux pendant 4 h.

L'excès du chlorure de thionyle et le benzène sont évaporés sous vide. Le résidu huileux 130 g est utilisé tel que pour la cyclisation.

4. On coule en 8 h la solution de 130 g du chlorure d'acide précédent dans 1500 ml de benzène dans une soltuion de 131 g de triéthylamine dans 1500 ml de benzène à la température ordinaire.

On abandonne 15 h à température ordinaire. Le mélange réactionnel est lavé à l'acide chlorhydrique normal puis à l'eau. La solution organique séchée est évaporée à sec sous vide. Le résidu 93 g huileux est filtré sur gel de silice dans le chlorure de méthylène.

La fraction de tête est évaporée et recristallisée dans l'hexane.

Rt=39,4 g F=55–58° MK

- la 2-trifluorométhyl 11-(méthoxycarbonylméthylidène) dibenzo (b,e) 1,4-dioxépine.
- la 2-trifluorométhyl 11-(méthoxycarbonylméthyl) [dibenzo (b,e) 1,4-dioxépine]
- la 2-trifluorométhyl 11-hydroxyméthyl [dibenzo (b,e) 1,4 dioxépine].
- la 2-trifluorométhyl 11-(p.toluène sulfonyloxy éthyl) dibenzo (b,e) 1,4-dioxépine.
- la N-diméthyl 2[2-trifluorométhyl dibenzo (b,e) 1,4-dioxépinyl-11] éthylamine
- la fumarate de N-diméthyl [2-trifluorométhyl dibenzo (b,e) 1,4-dioxépinyl-11] éthylamine.

Exemple 5

N-diéthyl 2-[dibenzo (b,e) 1,4-thioxépin-11-yl] éthylamine

En opérant selon le mode opératoire de l'exemple I au départ de l'acide (2'-hydroxyphényl) 2-thiobenzoique (F = 178 – 180°) on obtient successivement:

— la [dibenzo (b,e) 1,4-thioxépin] 11-one qui distille à 160 – 164° sous 0,05 mm Hg.
  PF = 128 – 130°
— le 11-(méthoxycarbonyl méthylidène) [dibenzo (b,e) 1,4-thioxépine]
  isomère A F = 126 – 130° (acétonitrile)
  isomère B F = 108 – 109° (éther isopropylique)
— la 11-(méthoxycarbonylméthyl) [dibenzo (b,e) 1,4-thioxépine]
  sous forme d'un produit huileux
— la 11-(p.toluénesulfonyloxyéthyl) dibenzo (b,e) 1,4-thioxépine
  T = 100 – 104° MK
  — la N-diéthyl 2[dibenzo (b,e) 1,4-thioxépin 11-yl] éthylamine
— le chlorhydrate de N-diéthyl 2[dibenzo (b,e) 1,4-thioxépin-11-yl] éthylamine

Exemple 6

dl 2-[dibenzo (b,e) 1,4-dioxépin-11-yl] éthyl 1-pyrrolidine et son chlorhydrate

Stade A

Acide dibenzo (b,e) 1,4-dioxépin-11-yl] méthylidène carboxylique (isomères Z et E)

On met en suspension dans 130 ml de méthanol 5,8 g de 11-(méthoxycarbonyl méthylidène) dibenzo (b,e) 1,4-dioxépine isomère B obtenu à l'exemple I stade B et on ajoute 26 ml d'hydroxyde de sodium à 40%. On laisse reposer le mélange pendant 12 heures à température ambiante puis on chasse le méthanol sous pression réduite. La solution limpide ainsi obtenue est diluée avec 50 ml d'eau et on épuise à l'éther. Après élimination de la phase éthérée on acidifie la phase aqueuse par l'acide chlorhydrique concentré tout en refroidissant à 0°. L'acide [dibenzo (b,e) 1,4-dioxépin 11'-yl]méthylidène carboxylique précipite. On le sépare, l'essore, le lave à l'eau et le sèche sous vide phosphorique. Rendement: 5 g d'un produit fondant à 174 – 180°.

Une nouvelle cristallisation dans l'acétate d'éthyle à chaud fornit 1,6 g d'un isomère pur à 185 – 195°. Le produit est entièrement soluble dans la soude N/10.

De la même façon, au départ de la 6,7 g de 11-(méthoxy carbonylméthylidène) dibenzo (b,e) 1,4-dioxépine (isomère A) on obitent 5,8 g d'acide [dibenzo (b,e) 1,4-dioxépin 11-yl] méthylidène carboxylique (isomère A) fondant à 186 – 190°.

Stade B

1[(dibenzo (b,e) 1,4-dioxépin 11-yl) méthylidène carbonyl] pyrrolidine

Dans un ballon à trois tubulures on introduit 1,2 g d'acide dibenzo (b,e) 1,4-dioxépin yl-11 méthylidène carboxylique et 10 ml d'acétone puis 1 g de triéthylamine. A la solution limpide on ajoute 0,46 g de chlurure de cyanuryle purifié (F = 144 – 145°). La réaction est exothermique et on maintient la température interne vers 15° à l'aide d'un bain d'eau glacée. On suit en CCM la disparition du Chlorure de cyanuryle. On ajoute alors 0,35 g de pyrrolidine et on laisse en contact pendant 1 heure 15 minutes.

La solution acétonique est évaporée à sec. Le résidu est repris au chlorure de méthylène. On lave la solution méthylénique à l'eau, à l'acide chlorhydrique normal; puis à la soude N et enfin à l'eau. On sèche cette solution puis l'évapore à sec. On obtient ainsi 1 g d'amide pyrrolidinique. Le résidu est purifié par chromatographie sur gel de silice et élution par un mélange acétate d'éthyle benzène. On recueille ainsi une fraction médiane fournissant 0,5 g de produit pur (F = 134 – 136°). On le recristallise de l'acétronitrile. Le point de fusion du produit pur est porté à 145 – 149°.

Stade C

1-[dibenzo(b,e) 1,4-dioxépin 11-yl] acétyl pyrrolidine

Dans une fiole conique on met en suspension 3,5 g de tournures d'aluminium préablement décapée

à l'acide chlorhydrique dans 20 ml d'éther puis on ajoute 1,7 g de chlorure mercurique. L'addition de chlorure mercurique provoque le reflux de solvant suivie de l'apparition d'un précipité puis on ajoute ensuite 6 g de 1 [dibenzo (b,e) 1,4-dioxépin-11 yl] méthylidène carbonyl pyrrolidine en solution dans 30 $cm^3$ de tétrahydrofuran sous agitation puis un mélange de 8 ml d'acétonitrile et de 1,58 ml d'eau en 3 heures environ. Au bout de ce délai, on sépare l'alumine qui s'est formée et on la rince avec quelques ml de tétrahydrofuran. On réunit les filtrats que l'on évapore à sec. Le résidu sec pèse 5,4 g.

Stade D

dl N2-[dibenzo (b,e) 1,4-dioxépin-11 yl] éthyl 1-pyrrolidine

Le résidu sec obtenu au stade précédent est mis en solution dans 45 ml d'acitate d'éthyle. On ajoute 0,5 g de charbon palladié à 5% et on procède à l'hydrogénation catalytique après purge du milieu par barbottage d'azote sous une pression de 50 bars. Après cessation d'absorption d'hydrogène, on sépare le catalyseur que l'on rince à plusieurs reprises à l'éthanol. On réunit les filtrats et on acidifie le milieu par de l'acide chlorhydrique gazeux. Le chlorhydrate précipite progressivement. On laisse reposer la suspension une nuit en glacière et on filtre le précipité. Les cristaux sont séchés puis recristallisés de l'acétonitrile.

Le chlorhydrate de dl 2[dibenzo (b,e) 1,4-dioxépin-11 yl] éthyl pyrrolidine fond à 191 – 197° (déc).

Le même produit peut être obtenu au départ de la 11-(p.toluène sulfonyloxyéthyl) dibenzo (b,e) 1,4-dioxépine par action de la pyrrolidine au reflux de diméthyl formamide. La base ainsi obtenue est ensuite convertie en chlorhydrate qui cristallise avec 0,33 mol d'eau. Le chlorhydrate fond à 195 – 197°.

Selon les procédés des exemplex précédents, les composés figurants dans le tableau ci-après ont été préparés:

| Ex | R | R' | $R_1$ | $R_2$ | A | Sel | P. F. |
|---|---|---|---|---|---|---|---|
| 7 | H | H | $CH_3$ | $CH_3$ | O | Fumarate | 145–147° MK |
| 8 | H | H | H | 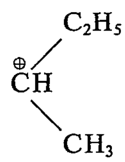 | O | Fumarate | 146–177° MK |
| 9 | H | H | H | t-butyl | O | Fumarate | 236–239° MK |
| 10 | H | H | H | $CH_3$ | O | Fumarate | 161–164° MK |
| 11 | H | H | H | i-propyl | O | Fumarate | 186–190° MK |
| 12 | H | H | $CH_3$ | $CH_3$ | S | Chlorhydrate | 205–215° MK |
| 13 | H | H | H | 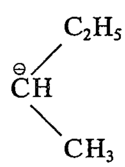 | O | Fumarate | 170–180° MK |
| 14 | Cl—2 | H | $CH_3$ | $CH_3$ | O | Fumarate | 156–160° MK |
| 15 | Cl—2 | H | $—(CH_2)_4—$ | $—(CH_2)_4—$ | O | Fumarate | 182–187° MK |
| 16 | H | H | $—(CH_2)_4—$ | $—(CH_2)_4—$ | S | Chlorhydrate | 190–196° MK |
| 17 | H | Cl—7 | $C_2H_5$ | $C_2H_5$ | O | Dichlorhydrate | 135–140° MK |

Etude pharmacologique des composés selon l'invention.

## 1. Test des convulsions par électrochocs

L'activité des composés selon l'invention a été mise en évidence sur des lots de souris.

Les souris sont protégées contre les convulsions provoquées par l'éctrochoc par des doses s+échelonnant selon les produits entre 1,5 mg et 10 mg/kg I. P. (dose efficace diminuant de 50% le nombre des convulsions). Dans les mêmes conditions le chlorazepate dipotassique provoque le même effet protecteur à la dose de 7 mg/kg.

## 2. Inhibition de l'agressivité

Ce test a été effectué sur des rats et des souris ayant préalablement subie l'ablation des bulbes olfactifs et soumis à l'isolement selon la méthode décrite par L. Valzelli; Aggresive behaviour 1969, p. 70 – 76 (Excerpta Medica Foundation-Amsterdam).

Aux doses de 10 et 20 mg/kg par voie intrapéritonéale en solution dans un solvant aqueux le nombre des animaux muricides ou agressifs est diminué respectivement de 35 et de 80%. L'effet inhibiteur sur l'agressivité des animaux ne s'accompagne d'aucun effet secondaire tel qu'hyperactivité ou état de dépression.

## 3. Antagonisme vis à vis de la tétrabénazine

Les composés selon l'invention, injectés à des lots de 10 souris aux doses de 10 et 25 mg/kg s. c., inhibent la ptose des paupières provoquée par injection de tétrabénazine. Pour la plupart des composés, l'inhibition est totalte à la dose de 25 mg/kg. Pour les plus actifs la dose de 10 mg/kg inhibe la ptose induite par la tétrabénazine. L'activité des composés selon l'invention sur ce test est au moins double de celle de la miansérine prise comme élément de référence.

## 4. Potentialisation des effets de l'amphétamine

L'injection intrapéritonéale de 5 mg/kg d'amphétamine à des lots de rats déclenche l'apparition des mouvements stéréotypés. Les composés selon l'invention administrés par voie orale à des doses s'échelonnant de 10 à 25 mg/kg trois heures avant l'injection d'amphétamine, potentialisent l'apparition et la durée de tels mouvements. Cet effet potentialisateur est comparable à celui de l'imipramine.

## 5. Antagonisme de l'hypothermie

Les composés selon l'invention, injectés par voie I. P. ou S. C. à la dose de 0,1 à 25 mg/kg chez la souris, antagonisent l'hypothermie provoquée par l'injection de 2,5 mg/kg de réserpine S. C. ou d'apomorphine de 15 mg/kg S. C.

## 6. Activité anticatatonique

Injectés à la dose de à 40 mg/kg I. P. chez le vat, les composés de l'invention inhibent la catatonie provoquée par le prochlorperazine. Cette inhibition est de l'ordre de celle provoquée par l'amitryptiline.

## 7. Détermination de la toxicité aigüe

Les composés selon l'invention ont été administrés par voie intrapéritonéale en solution dans l'eau à des doses croissantes s'échellonant de 20 à 200 mg/kg à des lots de 10 souris de souche Swiss pesant 20 g environ. Les animaux sont gardés 8 jours en observation. On dénombre les morts s'il y en a.

La dose léthale moyenne est calculée graphiequement selon la méthode de Tainter et Miller. Elle varie selon les composés entre 80 et 100 mg/kg. Par voie orale la DL 50 est de 250 à 500 mg/kg chez les souris et pour certains produits >500 mg/kg chez le rat.

**Revendications pour les Etats contractants: BE, CH, DE, GB, IT, LI, NL, SE**

1. Les dibenzooxépines substituées de formule générale I:

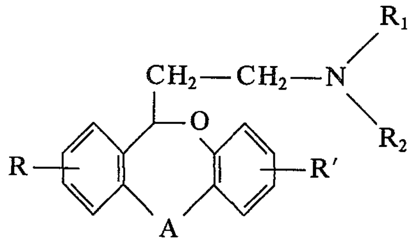

(I)

dans laquelle R et R', identiques ou différents, représentent un atome d'hydrogène ou d'halogène, ou bien un groupe trifluorométhyle,
A représente un atome d'oxygène ou un atome de soufre, et
$R_1$ et $R_2$, identiques ou différents, représentent chacun un atome d'hydrogène ou un groupe alkyle de 1 à 4 atomes de carbone, ou bien forment ensemble un groupe pyrrolidino, sous forme racémique ou d'isomères optiques,
ainsi que leurs sels d'addition avec un acide minéral ou organique.

2. La dl 2-[dibenzo (b,e) 1,4-dioxépin-11-yl]-éthyl 1-pyrrolidine, ses sels et ses isomères optiques.

3. La dl N-diéthyl 2-[dibenzo (b,e) 1,4-dioxépin-11-yl] éthylamine, ses sels et ses isomères optiques.

4. La dl N-diméthyl 2-[dibenzo (b,e) 1,4-dioxépine-11-yl] éthylamine, ses sels et ses isomères optiques.

5. La dl N-diéthyl 2-[2-chloro dibenzo (b,e) 1,4-dioxépin-11 · yl éthylamine, ses sels et ses isomères optiques.

6. La dl N-méthyl 2-[dibenzo (b,e) 1,4-dioxépin-11-yl] éthylamine, ses sels et ses isomères optiques.

7. La dl N-diméthyl 2-[2-trifluorométhyldibenzo (b,e) 1,4-dioxépin-11-yl] éthylamine, ses sels et ses isomères optiques.

8. La dl N-diméthyl 2-[dibenzo (b,e) 1,4 thioxépin-11-yl] éthylamine, ses sels et ses isomères optiques.

9. Les compositions pharmaceutiques renfermant à titre de principe actif au moins un composé selon les revendications 1 à 8, en association ou en mélange avec un excipient ou un véhicule inerte, on toxique, pharmaceutiquement acceptable.

10. Une composition pharmaceutique selon la revendication 9 dans laquelle la teneur en principe actif s'échelonne entre 1 et 10 mg par prise unitaire.

11. Un procédé de préparation des dibenzo-oxépines de formule générale I selon la revendication 1 caractérisé en ce que l'on soumet un acide (hydroxyphényl)-A-benzoïque de formule générale II de la planche de formules
dans laquelle les substituants R et R' ont les significations fournies précédémment
et A est un atome d'oxygène ou de soufre
à l'action d'un agent déshydratant tel que l'anhydride acétique ou à l'actiondechlorure de thionyle et à la cyclisation par une amine tertiaire pour former une dibenzo oxépinone de formule générale III de la planche de formules,
dans laquelle la signification des substituants R et R' demeure inchangée
que l'on soumet à l'action sélective d'un ylide d'(alcoxycarbonylméthyl) phosphonium de formule générale IV

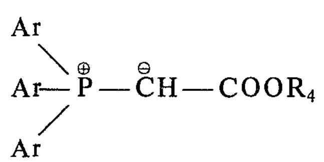

(IV)

dans laquelle Ar représente un radical phényle ou phényle substitué
et $R_4$ est un radical alcoyle inférieur ou aralcoyle, substitué ou non
pour former le dérivé (alcoxycarbonyl) méthylidénique correspondant de formule générale V de la planche de formules
dans laquelle les substituants A, R et R' gardent les significations fournies antérieurement
et $R_4$ est un radical alcoyle inférieur,
soumet ce dernier à l'action d'un agent d'hydrogénation pour former le dérivé (alcoxycarbonyl) méthylénique correspondant de formule générale VI de la planche de formules, dans laquelle les substituants R, R', $R_4$ et A sont définis comme précédemment puis réduit celui-ci en éthanol correspondant par action d'un hydrure mixte de métal alcalin pour obtenir un composé de formule générale VII de la planche de formules dans laquelle les substituants R, R' et A sont définis comme

précédemment et on soumet ce dérivé hydroxylé à l'action d'un agent d'estérification, pour obtenir l'ester correspondant de formule générale VIII de la planche de formules,
dans laquelle la définition des substituants R, R' et A demeure inchangée
et X est Cl, Br, I, alkyl$SO_3$ ou aryl$SO_3$
puis fait réagir celui-ci avec un dérivé aminé de formule générale IX

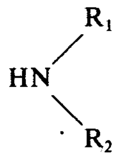
(IX)

dans laquelle les substituants $R_1$ et $R_2$ ont les définitions fournies précédemment
et obtient un dérivé éthylaminé de formule générale I
dans laquelle la définition des substituants R, R', $R_1$, $R_2$ et A demeure inchangée
que l'on peut
soit dédoubler en ses isomères optiques par salification à l'aide d'un acide optiquement-actif,
soit salifier par addition d'un acide minéral ou organique.

12. Procédé selon la revendication 11, caractérisé en ce que l'on soumet une dibenzo (b,e) oxépin 10-one de formule générale III de la planche de formules, dans laquelle les substituants R, R' et A ont les significations fournies précédemment
à l'action d'un ylide de cyanométhyl phosphonium de formule générale IV'

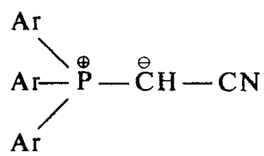
(IV')

dans laquelle Ar est défini comme précédemment
pour former un dérivé cyanométhylidénique de formule générale XI de la planche de formules, dans laquelle A, R et R' sont définis comme précédemment,
que l'on hydrogène sélectivement en dérivés aminoéthylidéniques correspondants de formule générale XII de la planche de formules, sous forme Z ou E
dans laquelle les substituants A, R et R' sont définis comme précédemment,
puis réduit ces derniers à l'aide d'un hydrure mixte de métal alcalin pour former un dérivé éthylaminé de formule générale XIII de la planche de formules
dans laquelle la définition des substituants A, R et R' demeure inchangée
que l'on peut, lorsque désiré
soit salifier par addition d'un acide minéral ou organique
soit dédoubler en ses isomères optiques par salification à l'aide d'unacide chiral
soit alcoyler ou aralcoyler par action d'un dérivé carbonylé en présence d'un agent réducteur.

13. Procédé selon la revendication 11, qui consite à soumettre un composé (alcoxycarbonyl) méthylidénique de formule générale V de la planche de formules à une saponification ménagée en milieu alcalin pour former un dérivé (hydroxycarbonyl) méthylidénique de formule générale XIV de la planche de formules, dans laquelle les substituants R, R' et A ont les signification fournies antérieurement,
sous forme Z ou E
que l'on condense avec un dérivé aminé de formule générale IX

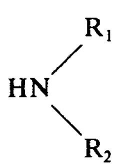
(IX)

dans laquelle les substituants $R_1$ et $R_2$ ont les significations fournies précédemment
en présence d'un agent de fonctionnalisation du carboxyle
pour former l'amide méthylidénique correspondante de formule générale XV de la planche de formules, sous forme Z ou E,
dans laquelle les substituants $R_1$, $R_2$, R, R' et A ont les significations fournies antérieurement,
que l'on peut réduire ou hydrogéner en un dérivé de formule générale I.

**Revendications pour l'Etat contractant: AT**

1. Procédé de préparation de dibenzooxépines substituées de formule générale I:

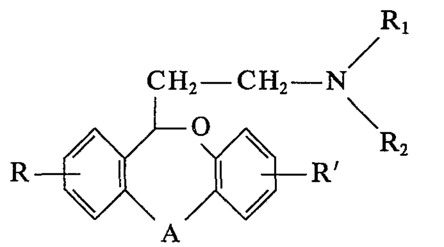

(I)

dans laquelle R et R', identiques ou différents, représentent un atome d'hydrogène ou d'halogène, ou bien un groupe trifluorométhyle,
A représente un atome d'oxygène ou un atome de soufre, et
$R_1$ et $R_2$, identiques ou différents, représentent chacun un atome d'hydrogène ou un groupe alkyle de 1 à 4 atomes de carbone, ou bien forment ensemble un groupe pyrrolidino, sous forme racémique ou d'isomères optiques,
ainsi que leurs sels d'addition avec un acide minéral ou organique, procédé caractérisé en ce que l'on soumet un acide (hydroxyphényl)-A-benzoïque de formule générale II de la planche de formules
dans laquelle les substituants R et R' ont les significations fournies précédemment
et A est un atome d'oxygène ou de soufre
à l'action d'un agent déshydratant tel que l'anhydride acétique ou à l'action de chlorure de thionyle et à la cyclisation par une amine tertiaire pour former une dibenzo oxépinone de formule générale III de la planche de formules,
dans laquelle la signification des substituants R et R' demeure inchangée
que l'on soumet à l'action sélective d'un ylide d'(alcoxycarbonylméthyl) phosphonium de formule générale IV

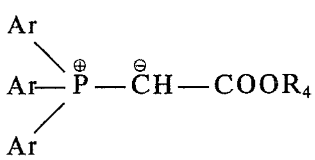

(IV)

dans laquelle Ar représente un radical phényle ou phényle substitué
et $R_4$ est un radical alcoyle inférieur ou aralcoyle, substitué ou non
pour former le dérivé (alcoxycarbonyl) méthylidénique correspondant de formule générale V de la planche de formules
dans laquelle les substituants A, R et R' gardent les significations fournies antérieurement
et $R_4$ est un radical alcoyle inférieur,
soumet ce dernier à l'action d'un agent d'hydrogénation pour former le dérive (alcoxycarbonyl) méthylénique correspondant de formule générale VI de la planche de formules, dans laquelle les substituants R, R', $R_4$ et A sont définis comme précédemment puis réduit celui-ci en éthanol correspondant par action d'un hydrure mixte de métal alcalin pour obtenir un composé de formule générale VII de la planche de formules dans laquelle les substituants R, R' et A sont définis comme précédemment et on soumet ce dérivé hydroxylé à l'action d'un agent d'estérification, pour obtenir l'ester correspondant de formule générale VIII de la planche de formules,
dans laquelle la définition des substituants R, R' et A demeure inchangée
et X est Cl, Br, I, alkyl$SO_3$ ou aryl$SO_3$
puis fait réagir celui-ci avec un dérivé aminé de formule générale IX

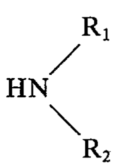

(IX)

dans laquelle les substituants $R_1$ et $R_2$ ont les définitions fournies précédemment
et obtient un dérivé éthylaminé de formule générale I
dans laquelle la définition des substituants R, R', $R_1$, $R_2$ et A demeure inchangée
que l'on peut
soit dédoubler en ses isomères optiques par salification à l'aide d'un acide optiquement-actif,
soit salifier par addition d'un acide minéral ou organique.

2. Procédé selon la revendication 1, caractérisé en ce que l'on soumet une dibenzo (b,e) oxépin 10-one de formule générale III de la planche de formules, dans laquelle les substituants R, R' et A ont les significations fournies précédemment
à l'action d'un ylide de cyanométhyl phosphonium de formule générale IV'

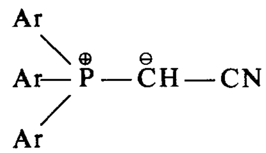
(IV')

dans laquelle Ar est défini comme précédemment
pour former un dérivé cyanométhylidénique de formule générale XI de la planche de formules, dans laquelle A, R et R' sont définis comme précédemment, que l'on hydrogène sélectivement en dérivés aminoéthylidéniques correspondants de formule générale XII de la planche de formules, sous forme Z ou E
dans laquelle les substituants A, R et R' sont définis comme précédemment,
puis réduit ces derniers à l'aide d'un hydrure mixte de métal alcalin pour former un dérivé éthylaminé de formule générale XIII de la planche de formules
dans laquelle la définition des substituants A, R et R' demeure inchangée
que l'on peut, lorsque désiré
soit salifier par addition d'un acide minéral ou organique
soit dédoubler en ses isomères optiques par salification à l'aide d'un acide chiral
soit alcoyler ou aralcoyler par action d'un dérivé carbonylé en présence d'un agent réducteur.

3. Procédé selon la revendication 2, qui consite à soumettre un composé (alcoxycarbonyl) méthylidénique de formule générale V de la planche de formules à une saponification ménagée en milieu alcalin pour former un dérivé (hydroxycarbonyl) méthylidénique de formule générale XIV de la planche de formules, dans laquelle les substituants R, R' et A ont les signification fournies antérieurement,
sous forme Z ou E
que l'on condense avec un dérivé aminé de formule générale IX

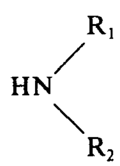
(IX)

dans laquelle les substituants $R_1$ et $R_2$ ont les significations fournies précédemment
en présence d'un agent de fonctionnalisation du carboxyle
pour former l'amide méthylidénique correspondante de formule générale XV de la planche de formules, sous forme Z ou E,
dans laquelle les substituants $R_1$, $R_2$, R, R' et A ont les significations fournies antérieurement,
que l'on peut réduire ou hydrogéner en un dérivé de formule générale I.

**Patentansprüche für die Vertragsstaaten: BE, CH, DE, GB, IT, LI, NL, SE**

1. Substituierte Dibenzooxepine der allgemeinen Formel I:

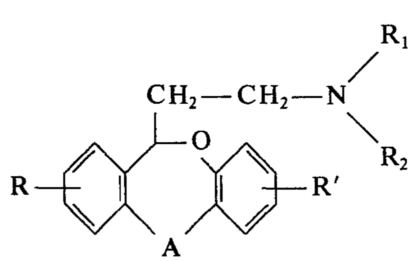
(I)

worin R und R' gleich oder verschieden sind und ein Wasserstoffatom oder ein Halogenatom darstellen, oder aber eine Trifluormethylgruppe,
A ein Sauerstoffatom oder ein Schwefelatom bedeutet und
$R_1$ und $R_2$ gleich oder verschieden sind und einzeln ein Wasserstoffatom oder eine Alkylgruppe mit 1 bis 4 Kohlenstoffatomen bedeuten, oder aber zusammen eine Pyrrolidino-Gruppe bilden,
in Form des Racemats oder der optisch aktiven Isomeren, sowie ihre Additionssalze mit einer

anorganischen oder organischen Säure.

2. D,L-2-[Dibenzo (b,e) 1,4-dioxepin-11-yl]-ethyl-1-pyrrolidin, seine Salze und optischen Isomeren.

3. D,L-N-Diethyl-2-[dibenzo (b,e) 1,4-dioxepin-11-yl]-ethylamin, seine Salze und optischen Isomeren.

4. D,L-N-Dimethyl-2-[dibenzo (b,e) 1,4-dioxepin-11-yl]-ethylamin, seine Salze und optischen Isomeren.

5. D,L-N-Diethyl-2-[2-chlor-dibenzo (b,e) 1,4-dioxepin-11-yl]-ethylamin, seine Salze und optischen Isomeren.

6. D,L-N-Methyl-2-[dibenzo (b,e) 1,4-dioxepin-11-yl]-ethylamin, seine Salze und optischen Isomeren.

7. D,L-N-Dimethyl-2-[2-Trifluormethyl-dibenzo (b,e) 1,4-dioxepin-11-yl]-ethylamin, seine Salze und optischen Isomeren.

8. D,L-N-Dimethyl-2-[Dibenzo (b,e) 1,4-thioxepin-11-yl]-ethylamin, seine Salze und optischen Isomeren.

9. Pharmazeutische Zusammensetzungen die als Wirkstoff mindestens eine Verbindung nach den Ansprüchen 1 bis 8 enthalten, in Verbindung oder in Mischung mit einem nicht-toxischen, pharmazeutisch verträglichen Bindemittel oder inerten Träger.

10. Pharmazeutische Zusammensetzung nach Anspruch 9, dadurch gekennzeichnet, daß sie einen Gehalt zwischen 1 und 10 mg Wirkstoff pro Einzeldosis enthält.

11. Verfahren zur Herstellung der Dibenzooxepine der allgemeinen Formel I nach Anspruch 1, dadurch gekennzeichnet, daß man eine (Hydroxyphenyl)-A-Benzoesäure der allgemeinen Formel II des Formelschemas,
worin die Substituenten R und R′ die vorstehend angegebene Bedeutung besitzen, und
A ein Sauerstoffatom oder ein Schwefelatom ist,
der Einwirkung eines dehydratisierenden Mittels wie Essigsäureanhydrid oder der Einwirkung von Thionylchlorid unterwirft, und einer Zyklisierung mit einem tertiären Amin zur Bildung eines Dibenzooxepinons der allgemeinen Formel III des Formelschemas,
worin die Bedeutung der Substituenten R und R′ unverändert ist,
welches man einer selektiven Einwirkung eines (Alkoxycarbonylmethyl)phosphoniumylids der allgemeinen Formel IV

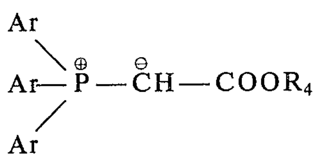

(IV)

worin Ar einen Phenylrest oder substituierten Phenylrest bedeutet, und
$R_4$ ein substituierter oder nicht-substituierter niederer Alkylrest oder Aralkylrest ist,
unterwirft zur Bildung eines entsprechenden (Alkoxycarbonyl)-Methyliden-Derivats der allgemeinen Formel V des Formelschemas,
worin die Substituenten A, R und R′ die vorstehend angegebenen Bedeutungen besitzen, und
$R_4$ ein niederer Alkylrest ist,
das letztere der Einwirkung eines hydrierenden Mittels unterwirft zur Bildung des entsprechenden (Alkoxycarbonyl)-Methylen-Derivats der allgemeinen Formel VI des Formelschemas, worin die Substituenten R, R′, $R_4$ und A die oben angegebene Bedeutung besitzen, dieses dann zum entsprechenden Ethanol durch Einwirkung einer mit einem Alkalimetall gemischten Wasserstoffverbindung reduziert zur Bildung einer Verbindung der allgemeinen Formel VII des Formelschemas, worin die Substituenten R, R′ und A die oben angegebene Bedeutung besitzen, und diese Hydroxylderivat der Einwirkung eines Veresterungsmittels unterwirft, um den entsprechenden Ester der allgemeinen Formel VIII des Formelschemas zu erhalten,
worin die Bedeutung des Substituenten R, R′ und A unverändert ist, und
X Cl, Br, J, $AlkylSO_3-$ oder $ArylSO_3-$ ist
diesen dann mit einem Aminderivat der allgemeinen Formel IX

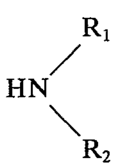

(IX)

worin die Substituenten $R_1$ und $R_2$ die oben angegebenen Bedeutungen besitzen, umsetzt,
und ein Ethylaminderivat der allgemeinen Formel I erhält, worin die Bedeutung des Substituenten R, R′, $R_1$, $R_2$ und A unverändert ist,
und wenn erwünscht, dieses in seine optischen Isomeren durch Salzbildung mittels einer optisch

aktiven Säure aufspaltet,
oder durch Zugabe einer anorganischen oder organischen Säure in ein Salz überführt.

12. Verfahren nach Anspruch 11, dadurch gekennzeichnet, daß man ein Dibenzo (b,e) oxepin-10-on der allgemeinen Formel III des Formelschemas, worin die Substituenten R, R' und A die vorstehend angegebenen Bedeutungen besitzen,
der Einwirkung eines Cyanomethylphosphoniumylids der allgemeinen Formel IV' unterwirft

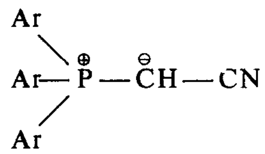

(IV')

worin Ar die oben angegebene Bedeutung besitzt,
zur Bildung eines Cyanomethylidenderivats der allgemeinen Formel XI des Formelschemas, worin A, R und R' die oben angegebene Bedeutung besitzen,
welches man selektiv in entsprechende Aminoethyliden-Derivate der allgemeinen Formel XII des Formelschemas, unter Form Z oder E, hydriert,
worin die Substituenten A, R und R' die oben angegebene Bedeutung besitzen, das letztere dann mit Hilfe einer Wasserstoffverbindung im Gemisch mit einem Alkalimetall zur Bildung eines Ethylamin-Derivats der allgemeinen Formel XIII des Formelschemas reduziert,
worin die Bedeutung des Substituenten A, R und R' unverändert ist,
und, wenn erwünscht, durch Zugabe einer anorganischen oder organischen Säure ein Salz bildet,
oder mittels einer optisch aktiven (chiralen) Säure in die optischen Isomeren aufspaltet,
oder durch Einwirkung eines Carbonylderivats in Gegenwart eines Reduktionsmittels alkyliert oder aralkyliert.

13. Verfahren nach Anspruch 11, dadurch gekennzeichnet, daß man eine (Alkoxycarbonyl)-Methylidenverbindung der allgemeinen Formel V des Formelschemas einer schonenden Verseifung in einem alkalischen Medium unterwirft zur Bildung eines (Hydroxycarbonyl)-Methyliden-Derivats der allgemeinen Formel XIV des Formelschemas, worin die Substituenten R, R' und A die vorstehend angegebene Bedeutung besitzen, unter Form Z oder E,
dieses mit einem Aminderivat der allgemeinen Formel IX

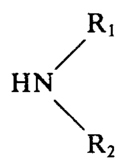

(IX)

worin die Substituenten $R_1$ und $R_2$ die oben angegebene Bedeutung besitzen, in Gegenwart eines Agens mit Carboxylfunktion kondensiert zur Bildung des entsprechenden Methylidenamids der allgemeinen Formel XV des Formelschemas, unter Form Z oder E,
worin die Substitutenten $R_1$, $R_2$, R, R' und A die vorstehend angegebenen Bedeutungen besitzen,
welches man zu einem Derivat der allgemeinen Formel I reduzieren oder hydrieren kann.

**Patentansprüche für den Vertragsstaat: AT**

1. Verfahren zur Herstellung der Dibenzooxepine der allgemeinen Formel I

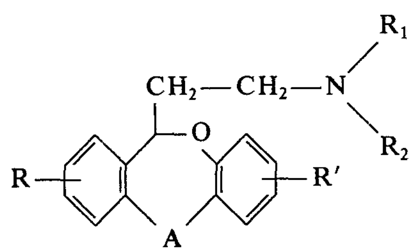

(I)

worin R und R' gleich oder verschieden sind und ein Wasserstoffatom oder ein Halogenatom darstellen, oder aber eine Trifluormethylgruppe,
A ein Sauerstoffatom oder ein Schwefelatom bedeutet und
$R_1$ und $R_2$ gleich oder verschieden sind und einzeln ein Wasserstoffatom oder eine Alkylgruppe mit 1 bis 4 Kohlenstoffatomen bedeuten, oder aber zusammen eine Pyrrolidino-Gruppe bilden,
in Form des Racemats oder der optisch aktiven Isomeren, sowie ihre Additionssalze mit einer

anorganischen oder organischen Säure,
dadurch gekennzeichnet, daß man eine (Hydroxy-phenyl)-A-Benzoesäure der allgemeinen Formel II des Formelschemas,
worin die Substituenten R und R′ die vorstehend angegebene Bedeutung besitzen, und
A ein Sauerstoffatom oder ein Schwefelatom ist,
der Einwirkung eines dehydratisierenden Mittels wie Essigsäureanhydrid oder der Einwirkung von Thionylchlorid unterwirft, und einer Zyklisierung mit einem tertiären Amin zur Bildung eines Dibenzooxepinons der allgemeinen Formel III des Formelschemas,
worin die Bedeutung der Substituenten R und R′ unverändert ist,
welches man einer selektiven Einwirkung eines (Alkoxycarbonylmethyl)phosphoniumylids der allgemeinen Formel IV

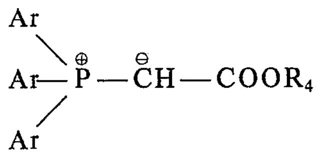

(IV)

worin Ar einen Phenylrest oder substituierten Phenylrest bedeutet, und
$R_4$ ein substituierter oder nicht-substituierter niederer Alkylrest oder Aralkylrest ist,
unterwirft zur Bildung eines entsprechenden (Alkoxycarbonyl)-Methyliden-Derivats der allgemeinen Formel V des Formelschemas,
worin die Substituenten A, R und R′ die vorstehend angegebenen Bedeutungen besitzen, und
$R_4$ ein niederer Alkylrest ist,
das letztere der Einwirkung eines hydrierenden Mittels unterwirft zur Bildung des entsprechenden (Alkoxycarbonyl)-Methylen-Derivats der allgemeinen Formel VI des Formelschemas, worin die Substituenten R, R′, $R_4$ und A die oben angegebene Bedeutung besitzen, dieses dann zum entsprechenden Ethanol durch Einwirkung einer mit einem Alkalimetall gemischten Wasserstoffverbindung reduziert zur Bildung einer Verbindung der allgemeinen Formel VII des Formelschemas, worin die Substituenten R, R′ und A die oben angegebene Bedeutung besitzen, und diese Hydroxylderivat der Einwirkung eines Veresterungsmittels unterwirft, um den entsprechenden Ester der allgemeinen Formel VIII des Formelschemas zu erhalten,
worin die Bedeutung des Substituenten R, R′ und A unverändert ist, und
X Cl, Br, J, $AlkylSO_3-$ oder $ArylSO_3-$ ist
diesen dann mit einem Aminderivat der allgemeinen Formel IX

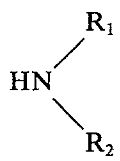

(IX)

worin die Substituenten $R_1$ und $R_2$ die oben angegebenen Bedeutungen besitzen, umsetzt,
und ein Ethylaminderivat der allgemeinen Formel I erhält, worin die Bedeutung des Substituenten R, R′, $R_1$, $R_2$ und A unverändert ist,
und wenn erwünscht, dieses in seine optischen Isomeren durch Salzbildung mittels einer optisch aktiven Säure aufspaltet,
oder durch Zugabe einer anorganischen oder organischen Säure in ein Salz überführt.

2. Verfahren nach Anspruch 1, dadurch gekennzeichnet, daß man ein Dibenzo (b,e) oxepin-10-on der allgemeinen Formel III des Formelschemas, worin die Substituenten R, R′ und A die vorstehend angegebenen Bedeutungen besitzen,
der Einwirkung eines Cyanomethylphosphoniumylids der allgemeinen Formel IV′ unterwirft

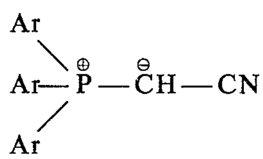

(IV′)

worin Ar die oben angegebene Bedeutung besitzt,
zur Bildung eines Cyanomethylidenderivats der allgemeinen Formel XI des Formelschemas, worin A, R und R′ die oben angegebene Bedeutung besitzen,
welches man selektiv in entsprechende Aminoethyliden-Derivate der allgemeinen Formel XII des Formelschemas, unter Form Z oder E, hydriert,
worin die Substituenten A, R und R′ die oben angegebene Bedeutung besitzen, das letztere dann mit

Hilfe einer Wasserstoffverbindung im Gemisch mit einem Alkalimetall zur Bildung eines Ethylamin-Derivats der allgemeinen Formel XIII des Formelschemas reduziert,
worin die Bedeutung des Substituenten A, R und R′ unverändert ist,
und, wenn erwünscht, durch Zugabe einer anorganischen oder organischen Säure ein Salz bildet,
oder mittels einer optisch aktiven (chiralen) Säure in die optischen Isomeren aufspaltet,
oder durch Einwirkung eines Carbonylderivats in Gegenwart eines Reduktionsmittels alkyliert oder aralkyliert.

3. Verfahren nach Anspruch 2, dadurch gekennzeichnet, daß man eine (Alkoxycarbonyl)-Methylidenverbindung der allgemeinen Formel V des Formelschemas einer schonenden Verseifung in einem alkalischen Medium unterwirft zur Bildung eines (Hydroxycarbonyl)-Methyliden-Derivats der allgemeinen Formel XIV des Formelschemas, worin die Substituenten R, R′ und A die vorstehend angegebene Bedeutung besitzen, unter Form Z oder E,
dieses mit einem Aminderivat der allgemeinen Formel IX

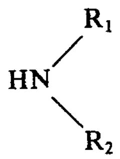

(IX)

worin die Substituenten $R_1$ und $R_2$ die oben angegebene Bedeutung besitzen, in Gegenwart eines Agens mit Carboxylfunktion kondensiert zur Bildung des entsprechenden Methylidenamids der allgemeinen Formel XV des Formelschemas, unter Form Z oder E,
worin die Substituenten $R_1$, $R_2$, R, R′ und A die vorstehend angegebenen Bedeutungen besitzen,
welches man zu einem Derivat der allgemeinen Formel I reduzieren oder hydrieren kann.

**Claims for the Contracting States: BE, CH, DE, GB, IT, LI, NL, SE**

1. Substituted dibenzooxepines of general formula I:

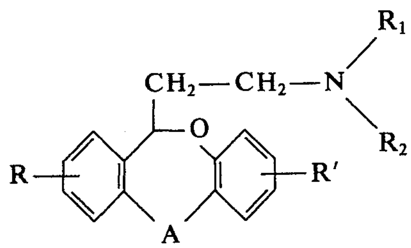

(I)

wherein R and R′, the same or different, are a hydrogen or a halogen atom, or a trifluoromethyl group,
A represents an oxygen or a sulphur atom,
$R_1$ and $R_2$, which may be the same or different, each represent a hydrogen atom, an alkyl radical of 1 to 4 carbon atoms, or may form together a pyrrolidino group,
in the racemic form or as optical isomers,
and their addition salts with a mineral or organic acid.

2. dl 2-[dibenzo (b,e)-1,4-dioxepin-11-yl]-ethyl 1-pyrrolidine, its salts and optical isomers.

3. dl N-diethyl 2-[dibenzo (b,e)-1,4-dioxepin-11-yl] ethylamine, its salts and optical isomers.

4. dl N-dimethyl 2-[dibenzo (b,e)-1,4-dioxepin-11-yl] ethylamine, its salts and optical isomers.

5. dl N-diethyl 2-[2-chloro dibenzo (b,e)-1,4-dioxepin-11-yl] ethylamine, its salts and optical isomers.

6. dl N-methyl 2-[dibenzo (b,e)-1,4-dioxepin-11-yl] ethylamine, its salts and optical isomers.

7. dl N-dimethyl 2-[2-trifluoromethyldibenzo (b,e)-1,4-dioxepin-11-yl] ethylamine, its salts and optical isomers.

8. dl N-dimethyl 2-[dibenzo (b,e)-1,4-thioxepin-11-yl] ethylamine, its salts and optical isomers.

9. Pharmaceutical compositions comprising as active ingredient at least one compound as claimed in Claims 1 to 8 in admixture or conjunction with an inert, non-toxic pharmaceutically acceptable carrier or vehicle.

10. A pharmaceutical composition as claimed in Claim 9, wherein the active principle is present at a unit dosage of between 1 and 10 mg.

11. A process for producing dibenzooxepines of general formula I as claimed in Claim 1, charactetrised by submitting a hydroxyphenyl-A-benzoic acid of general formula II of the formulae drawings,
in which R and R′ have the previously given meanings
and A is an oxygen or a sulphur atom,
to the action of a deshydrating agent such as acetic anhydride or to the action of thionyl chloride and

the cyclisation by a tertiary amine in order to obtain a dibenzooxepinone of general formula III of the formulae drawings, in which the meanings of R and R′ are as above defined, which is selectively submitted to the action of an ylide of (alkoxycarbonylmethyl)-phosphonium of general formula IV:

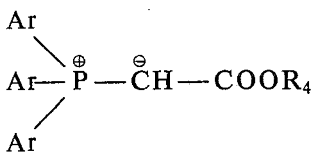

(IV)

wherein Ar represents a phenyl radical, optionally substituted and $R_4$ is a lower alkyl or aralkyl radical which may be substituted, in order to obtain the corresponding (alkoxycarbonyl)-methylidene derivative of general formula V of the formulae drawings in which A, R and R′ are as defined above and $R_4$ is a lower alkyl, and submit it to the action of a hydrogenating agent in order to obtain an alkoxycarbonyl methylenic derivative of general formula VI of the formulae drawings, in which R, R′, $R_4$ and A are the same as above, and which is then reduced in the corresponding ethanol by a mixed hydride of an alkali metal, to obtain a compound of general formula VII of the formulae drawing, in which R, R′ and A are as above defined and this hydroxy derivative is submitted to the action of an esterifying agent so as to obtain the corresponding ester of the general formula VIII of the formulae drawings, in which R, R′ and A are as defined above and X is Cl, Br, I, alkyl-$SO_3$ or aryl-$SO_3$ which is reacted with an amine derivative of general formula IX:

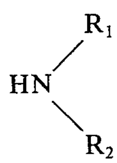

(IX)

in which $R_1$ and $R_2$ are as defined above, and an ethylamine derivative of general formula I is obtained, which is either separated in its optical isomers by salification using an optically active acid, or salified by the addition of a mineral or organic acid.

12. A process according to Claim 11, which consists in submitting a dibenzo (b,e)oxepin-10-one of general formula III of the formulae drawings in which R, R′ and A are defined as above, to the action of a cyanomethyl phosphonium-ylide of general formula IV′:

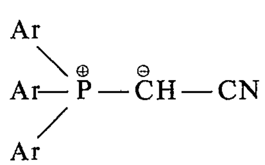

(IV′)

in which Ar is as previously defined, in order to obtain the cyanomethyllidenic derivative of genral formula XI of the formulae drawings, in which A, R and R′ are as previously defined, and which is selectively hydrogenated to form the corresponding amino-ethylidenic derivatives of general formula XII of the formulae drawings, in the form Z or E, in which A, R and R′ are as defined above, and which are reduced by means of a mixed hydride of an alkali metal to obtain an ethylamine derivative of general formula XIII of the formulae drawings in which A, R and R′ are as defined above, which may be, if desired, either salified by addition of a mineral or organic acid, or resolved in optical isomers by salification using a chilaric acid, or alkylated or aralkylated by a carbonylated derivative in the presence of a reducing agent.

13. A process according to Claim 11, which consists in submitting an (alkyloxycarbonyl) methylidenic compound of general formula V of the formulae drawings to a mild saponification in a basic medium to obtain a (hydroxycarbonyl) methylidenic derivative of general formula XIV of the formulae drawings, in which R, R′ and A have the previously given meanings,

in the form Z or E, which is condensed with an amine derivative of general formula IX

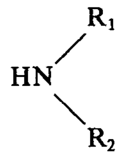

(IX)

wherein $R_1$ and $R_2$ are defined above, in the presence of a functionalisation agent for the carboxyl to obtain the corresponding methylidenic amide of general formula XV of the formulae drawings, in the form Z or E, wherein $R_1$, $R_2$, R, R′ and A are as previously defined, which may be reduced or hydrogenated in an ethylamine derivative of general formula I.

**Claims for the Contracting State: AT**

1. A process for producing dibenzooxepines of general formula I

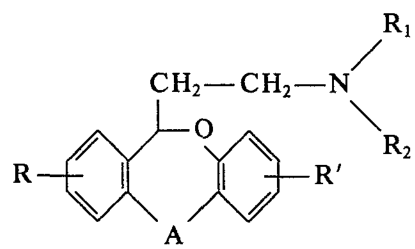

(I)

wherein R and R′, the same or different, are a hydrogen or a halogen atom, or a trifluoromethyl group, A represents an oxygen or a sulphur atom, $R_1$ and $R_2$, which may be the same or different, each represent a hydrogen atom, an alkyl radical of 1 to 4 carbon atoms, or may form together a pyrrolidino group, in the racemic form or as optical isomers, and their addition salts with a mineral or organic acid, characterised by submitting a hydroxyphenyl-A-benzoic acid of general formula II of the formulae drawings, in which R and R′ have the previously given meanings and A is an oxygen or a sulphur atom, to the action of a deshydrating agent such as acetic anhydride or to the action of thionyl chloride and the cyclisation by a tertiary amine in order to obtain a dibenzooxepinone of general formula III of the formulae drawings, in which the meanings of R and R′ are as above defined, which is selectively submitted to the action of an ylide of (alkoxycarbonylmethyl)-phosphonium of general formula IV:

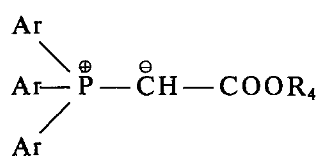

(IV)

wherein Ar represents a phenyl radical, optionally substituted and $R_4$ is a lower alkyl or aralkyl radical which may be substituted, in order to obtain the corresponding (alkoxycarbonyl)-methylidene derivative of general formula V of the formulae drawings in which A, R and R′ are as defined above and $R_4$ is a lower alkyl, and submit it to the action of a hydrogenating agent in order to obtain an alkoxycarbonyl methylenic derivative of general formula VI of the formulae drawings, in which R, R′, $R_4$ and A are the same as above, and which is then reduced in the corresponding ethanol by a mixed hydride of an alkali metal, to obtain a compound of general formula VII of the formulae drawings, in which R, R′ and A are as above defined and this hydroxy derivative is submitted to the action of an esterifying agent so as to obtain the corresponding ester of the general formula VIII of the formulae drawings,

in which R, R′ and A are as defined above
and X is Cl, Br, I, alkyl-$SO_3$ or aryl-$SO_3$
which is reacted with an amine derivative of general formula IX:

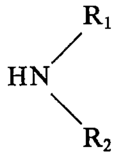

(IX)

in which $R_1$ and $R_2$ are as defined above,
and an ethylamine derivative of general formula I is obtained, which is
either separated in its optical isomers by salification using an optically active acid,
or salified by the addition of a mineral or organic acid.

2. A process according to Claim 1, which consits in submitting a dibenzo (b,e) oxepin-10-one of general formula III of the formulae drawings in which R, R′ and A are defined as above, to the action of a cyanomethyl phosphonium-ylide of general formula IV′:

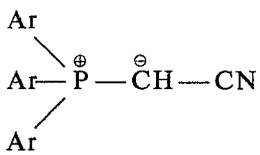

(IV′)

in which Ar ist as previously defined,
in order to obtain the cyanomethylidenic derivative of general formula XI of the formulae drawings,
in which A, R and R′ are as previously defined,
and which is selectively hydrogenated to form the corresponding amino-ethylidenic derivatives of general formula XII of the formulae drawings, in the form Z or E,
in which A, R and R′ are as defined above,
and which are reduced by means of a mixed hydride of an alkali metal to obtain an ethylamine derivative of general formula XIII of the formulae drawings in which A, R and R′ are as defined above,
which may be, if desired,
either salified by addition of a mineral or organic acid,
or resolved in optical isomers by salification using a chiralic acid,
or alkylated or arakylated by a carbonylated derivative in the presence of a reducing agent.

3. A process according to Claim 2, which consists in submitting an (alkyloxycarbonyl) methylidenic compound of general formula V of the formulae drawings to a mild saponification in a basic medium to obtain a (hydroxycarbonyl) methylidenic derivative of general formula XIV of the formulae drawings,
in which R, R′ and A have the previously given meanings,
in the form Z or E,
which is condensed with an amine derivative of general formula IX

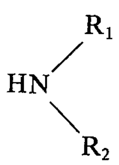

(IX)

wherein $R_1$ and $R_2$ are defined above,
in the presence of a functionalisation agent for the carboxyl to obtain the corresponding methylidenic amide of general formula XV of the formulae drawings,
in the form Z or E,
wherein $R_1$, $R_2$, R, R′ and A are as previously defined,
which may be reduced or hydrogenated in an ethylamine derivative of general formula I.

SCHEMA DE SYNTHESE

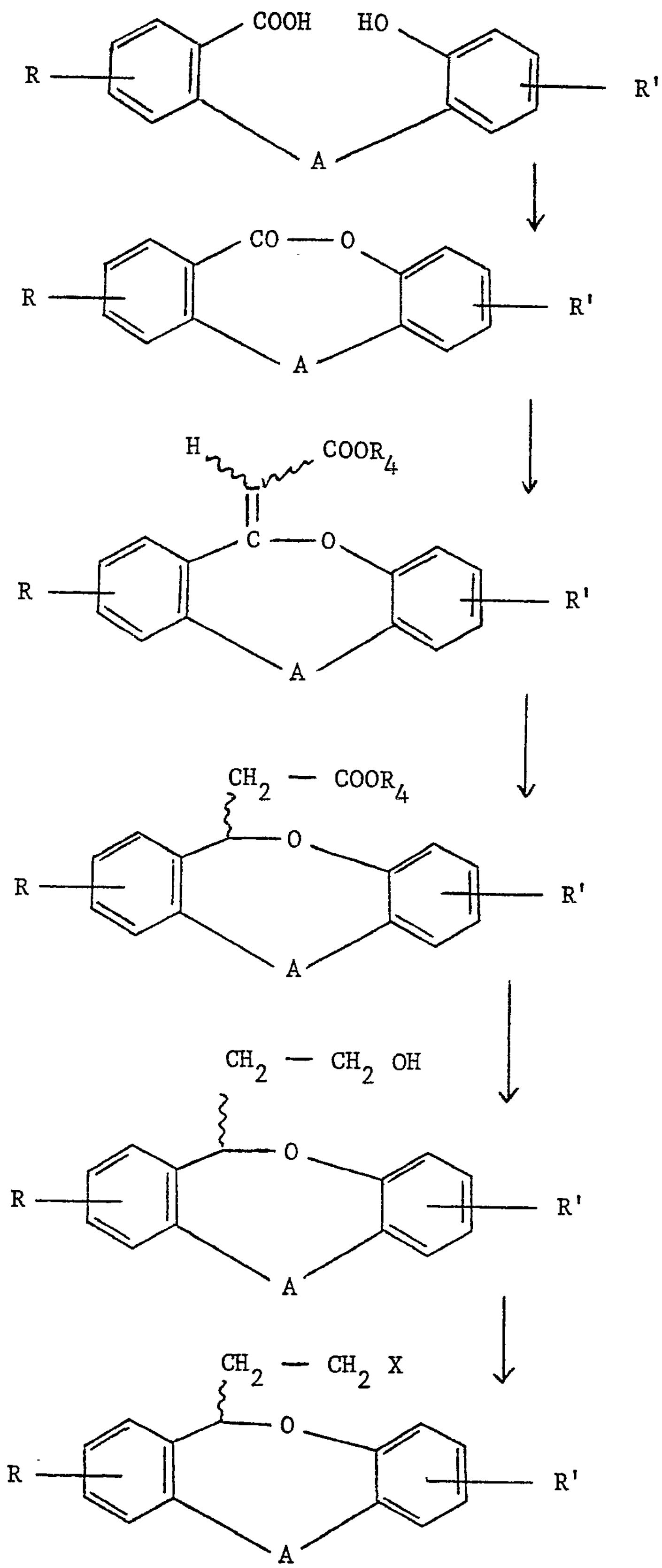

(II)

(III)

(V)

(VI)

(VII)

(VIII)

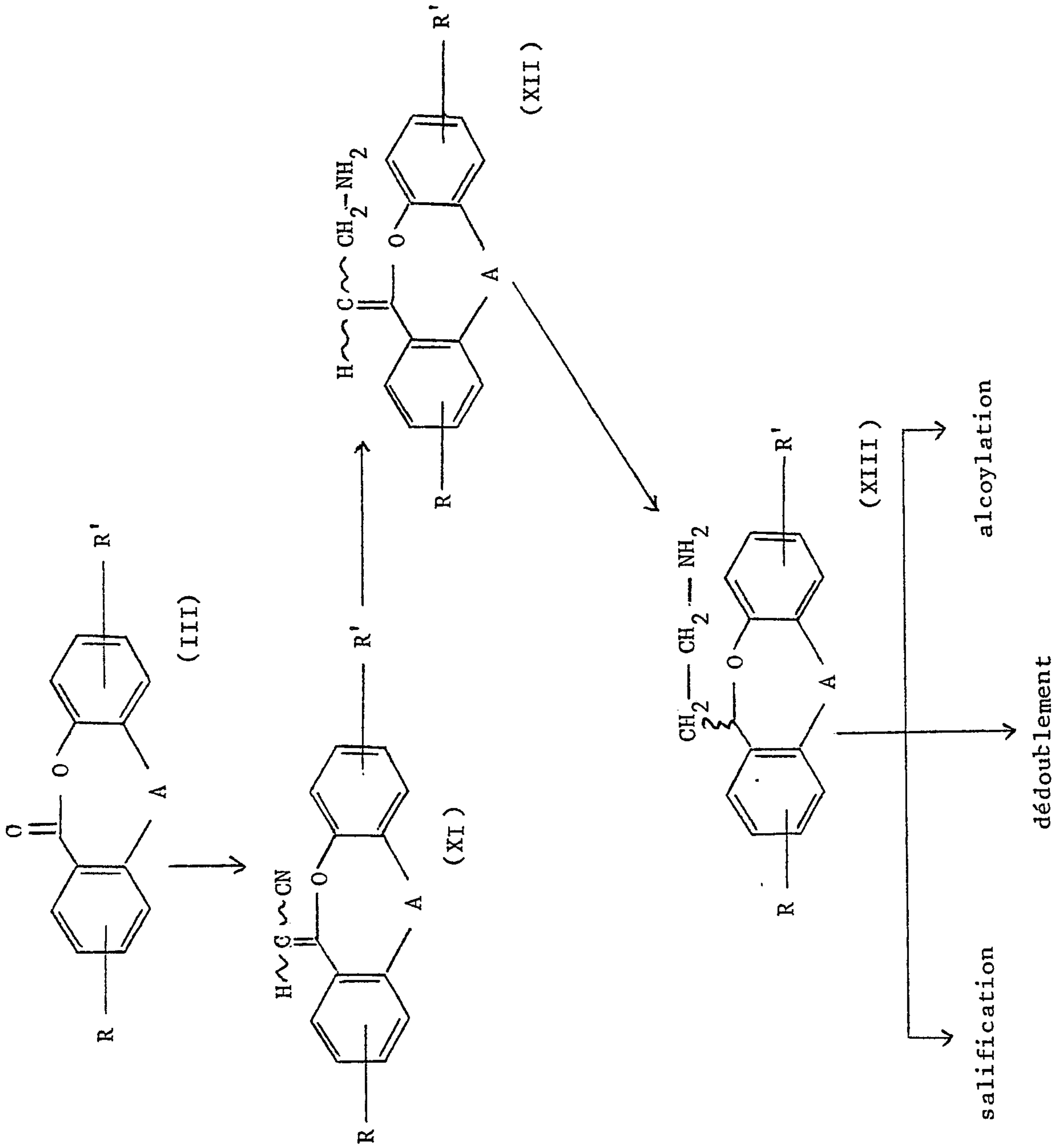


(III)

(XI)

(XII)

(XIII)

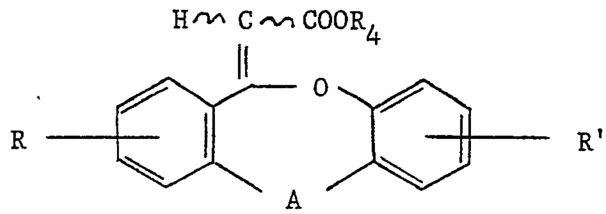

(V)

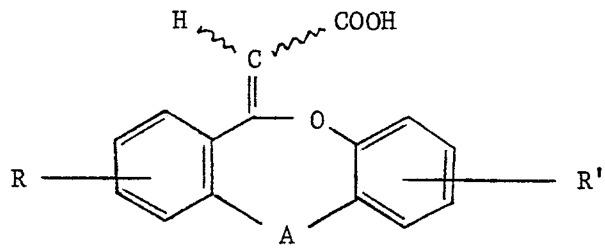

(XIV)

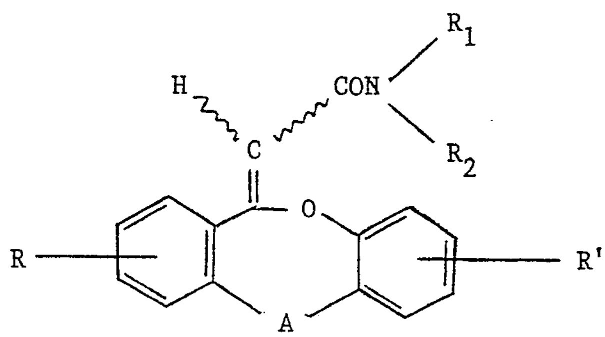

(XV)

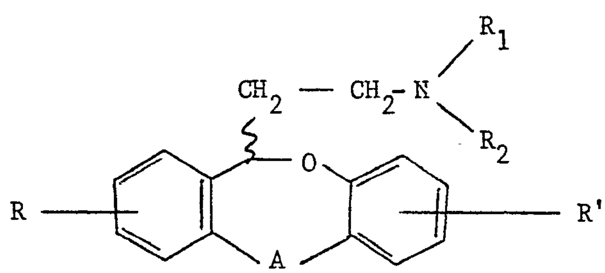

(I)